# FASCICULE DE BREVET EUROPEEN

(11) **EP 1 282 395 B1**
(45) Date de publication et mention de la délivrance du brevet: **06.10.2004**
(21) Numéro de dépôt: 01936504.8
(22) Date de dépôt: 14.05.2001
(51) Int. Cl.: A61K 7/40, A61K 7/48

(54) **UTILISATION DE L'ACIDE ELLAGIQUE COMME AGENT COSMETIQUE ANTI-POLLUTION**
VERWENDUNG VON ELLAGSÄURE ZUM SCHUTZ GEGEN VERSCHMUTZUNG
USE OF ELLAGIC ACID AS ANTI-POLLUTION COSMETIC AGENT

(30) Priorité: 18.05.2000 FR 0006384
(43) Date de publication de la demande: 12.02.2003
(73) Titulaire: L'OREAL, 75008 Paris (FR)
(72) Inventeur: DUCHE, Daniel, F-75013 Paris (FR); COTOVIO, José, F-77270 Dammartin-en-Go[le (FR); CATROUX, Philippe, F-94130 Nogent-sur-Marne (FR)
(74) Mandataire: Casalonga, Axel
(86) Numéro de dépôt international: PCT/FR2001/001454
(87) Numéro de publication internationale: WO 2001/087254

(56) Documents cités:
- EP-A- 0 496 173
- EP-A- 0 557 042
- EP-A- 0 914 815
- WO-A-00/48551
- FR-A- 2 768 927
- CHEMICAL ABSTRACTS, vol. 132, no. 15, 3 avril 2000 (2000-04-03) Columbus, Ohio, US; abstract no. 190636b, S. AHNED ET AL: "Ellagic acid ameliorates nickel induced biochemical alterations: diminution of oxidative stress" page 177; XP002161613 & HUM. EXP. TOXICOL., vol. 18, no. 11, 1999, pages 691-698,
- PATENT ABSTRACTS OF JAPAN vol. 014, no. 544 (C-0784), 4 décembre 1990 (1990-12-04) & JP 02 231407 A (LION CORP), 13 septembre 1990 (1990-09-13)
- PATENT ABSTRACTS OF JAPAN vol. 014, no. 544 (C-0784), 4 décembre 1990 (1990-12-04) & JP 02 231423 A (LION CORP), 13 septembre 1990 (1990-09-13)
- CHEMICAL ABSTRACTS, vol. 132, no. 23, 5 juin 2000 (2000-06-05) Columbus, Ohio, US; abstract no. 303114a, AMITA KAUL ET AL: "Plant polyphenols inhibit benzoyl peroxide-induced superoxide anion radical production and diacylglyceride formation in murine peritoneal macrophages" page 48; XP002161612 & NUTR. CANCER, vol. 35, no. 2, 1999, pages 207-211,

## Description

La présente invention concerne essentiellement une nouvelle utilisation de l'acide ellagique, de ses sels, de ses complexes métalliques, de ses dérivés mono- ou poly-éthers, mono- ou poly-acylés et de ses dérivés carbonates ou carbamates dérivant des groupements hydroxyles comme agent cosmétique anti-pollution.

Différents types de produits chimiques, xénobiotiques et particules composent la pollution urbaine.

Trois grandes catégories de polluants peuvent exercer des effets délétères sur la peau et le cheveu: les gaz, les métaux lourds et les particules qui sont les résidus de combustion sur lesquelles sont adsorbés de très nombreux composés organiques.

Ce sont les tissus les plus externes qui sont initialement et directement exposés aux toxiques de l'environnement. La peau est directement et fréquemment exposée à l'environnement pro-oxydant. Les sources d'oxydants environnementaux incluent l'oxygène, l'irradiation solaire UV ainsi que dans l'air pollué, l'ozone et les oxydes d'azote et de soufre. Les polluants atmosphériques représentés par les produits primaires et secondaires des combustions domestiques et industrielles tels que les hydrocarbures aromatiques mono- et polycycliques sont aussi une source importante du stress oxydatif. La peau est particulièrement sensible à l'action du stress oxydatif et la couche la plus externe sert de barrière vis-à-vis des dommages oxydatifs. Dans la plupart des circonstances, l'oxydant a des chances d'être neutralisé après réaction avec les matières kératiniques, mais les produits de réaction formés peuvent être responsables d'atteintes cellulaires et tissulaires.

Le stratum cornéum, la barrière de la peau, est le site de contact entre air et tissu cutané. La structure biphasique lipides/protéines est un déterminant crucial de cette fonction de barrière de la peau. Ces éléments peuvent réagir avec les oxydants et être altérés ce qui favorisera les phénomènes de desquamation. La peroxydation lipidique induite par l'ozone peut altérer la peau de deux façons:
1/ l'oxydation et la dégradation des lipides du stratum cornéum peut altérer la fonction barrière du stratum cornéum. La perturbation des lipides externes et de l'architecture des protéines semblent être des facteurs déclenchants dans de nombreuses dermatoses (psoriasis, dermatite atopique, dermatite irritante).
2/ la formation accrue de produits d'oxydation des lipides dans les couches de peau supérieures peut déclencher des atteintes dans les couches cutanées adjacentes. La réaction de l'ozone (O₃) avec les lipides insaturés implique des réactions d'addition sur les doubles liaisons. Ce pròcessus conduit dans un deuxième temps à la cassure des chaînes lipidiques et à la formation d'hydroperoxydes d'aldéhydes et de peroxyde d'hydrogène. Il s'agit d'un mécanisme spécifique différent du mécanisme de lipoperoxydation classiquement décrit, lequel est médié par un radical. Les produits d'oxydation lipidique secondaires ou tertiaires induits par l'ozone, lesquels ont une réactivité inférieure à l'ozone mais une durée de vie supérieure, peuvent propager l'effet de l'ozone. Du fait de leur stabilité relative, les produits d'oxydation des lipides et de peroxydation, c'est à dire les aldéhydes et les oxydes de cholestérol, ont le potentiel d'altérer les cellules à des sites distants non directement exposés à O₃.

Une atteinte oxydative significative au niveau des couches superficielles du stratum peut initier des processus inflammatoires localisés sous-jacents, conduisant au recrutement de phagocytes qui en générant des oxydants vont amplifier les processus oxydatifs initiaux.

Dans la pollution urbaine, l'exposition concomitante à O₃ et aux UV peut causer un stress oxydatif synergique.

De même, on peut penser qu'il existe une synergie d'action entre ozone et composés organiques issus de la combustion.

Une autre catégorie de polluants est constituée par les métaux lourds.

Les ions métalliques sont nécessaires à l'organisme sous forme de traces comme nutriments essentiels. Par exemple, plusieurs fonctions impliquant des polypeptides telles que des fonctions enzymatiques, structurales et immunologiques requièrent des cofacteurs métalliques.

Cependant, d'autres ions métalliques, en particulier les ions des métaux lourds lorsqu'ils sont à des concentrations non physiologique, peuvent altérer ces fonctions. Ainsi, la surexposition aux métaux de l'environnement peut conduire à des effets toxiques.

Des études écologiques conduites dans des pays industrialisés montrent que les quantités de métaux présents dans l'atmosphère sont croissantes. Ceci conduit à une augmentation des niveaux de métaux lourds dans les tissus des organismes consécutivement à l'ingestion de nourritures contaminées et à l'exposition aux métaux de l'atmosphère.

Les effets de l'accumulation des métaux lourds peuvent être extrêmement dangereux et leur toxicité est dûe en partie à l'altération des structures tertiaires et quaternaires des protéines, ce qui conduit à une réduction de leur activité catalytique. Les protéines altérées peuvent devenir antigéniques et entraîner une réponse immunitaire.

De plus, l'accumulation des métaux à partir de particules polluantes présentes dans l'air telles que le zinc, le cuivre, le cobalt, le manganèse, le mercure ou le nickel, développe chez les enfants des troubles de la mémoire.

Un autre mécanisme responsable des effets toxiques des métaux est la substitution compétitive de cofacteurs physiologiques naturels par les métaux lourds à concentrations non physiologiques. Ainsi, le contrôle des métaux lourds polluants dans l'atmosphère est essentiel pour prévenir des maladies en relation avec l'exposition aux métaux.

Du fait de la contamination croissante de l'environnement par les métaux lourds et de leur présence ubiquitaire dans l'écosystème, la peau, le cheveu et les muqueuses accessibles représentent la surface de contact la plus large et favorisent donc l'accumulation des métaux et leur absorption ultérieure dans l'organisme.

Certains métaux et composés métalliques présents dans les fabrications industrielles, les produits chimiques, la bijouterie, les vêtements, les produits médicamenteux, les colorants et les produits d'entretien sont impliqués dans des réactions d'irritation primaire, des réactions d'allergie et de carcinogénicité au niveau du tissu cutané.

Les métaux particulièrement incriminés dans l'environnement sont le cuivre, le cobalt, le zinc, le manganèse, le mercure, le nickel et le plomb.

Le rash cutané causé par la dermatite aux métaux est un problème rencontré chez les gens exposés à des quantités élevées de certains ions métalliques. L'exposition au nickel dans l'environnement est largement dû à l'usage fréquent de ce métal dans les articles les bijoux, les bracelets de montre et les boutons de vêtement. La sensibilisation au nickel avec le développement de dermatite est un hasard industriel dans certains métiers. Le dépôt de métaux sur le cheveu est un phénomène inévitable.

Le cheveu est un absorbant fort pour les métaux. La fixation est tellement forte qu'une fois ces métaux fixés capturés par les sites anioniques de la fibre, ils sont difficiles à extraire. Le degré de fixation des métaux sur le cheveu dépend généralement de plusieurs facteurs tels que la taille de la fibre, sa porosité, et le temps d'exposition . Les métaux comme le cuivre, le plomb et le fer peuvent interférer avec des traitements chimiques tels que la coloration et le permanentage du cheveu.

Les cheveux sont également des sites privilégiés par ces particules de métaux lourds. En effet, les fibres kératiniques possèdent des sites anioniques qui vont lier les métaux lourds cationiques et les accumuler. Certains produits cosmétiques contiennent des métaux comme le magnésium, le cuivre ou le fer. L'absorption de ces métaux par les fibres kératiniques peut interférer avec des traitements chimiques comme les colorations, le blanchiment ou les effets de permanente. Ces interactions peuvent conduire à des problèmes de coloration ou des précipitations comme décrit dans le brevet américain US-5 635 167.

Il a été démontré que certains métaux lourds pénètrent dans la peau et s'accumulent (A.B.G. Landsdown. Critical Reviews in Toxicology. 1995, 25:397-462). A forte concentration, ils peuvent induire: des mécanismes d'oxydation sur les lipides membranaires, une cytotoxicité directe, capable d'aboutir à une nécrose cellulaire et une alkylation des nucléophiles cellulaires pouvant être à l'origine des phénomènes de sensibilisation ou de carcinogénèse.

Une autre catégorie majeure de polluants est constituée par des résidus de combustions sous forme de particules sur lesquelles sont adsorbés de très nombreux composés organiques, et en particulier les hydrocarbures aromatiques polycycliques (HAP). Ces hydrocarbures aromatiques polycycliques adsorbés à la surface des particules et des poussières véhiculées par l'air urbain, peuvent pénétrer le tissu cutané et y être biotransformés. Leur métabolisme hépatique, bien décrit dans la littérature, conduit à des formations de métabolites monohydroxylés (voie de détoxification), d'époxydes et de diols époxydes (voie toxifiante). On peut observer des phénomènes similaires au niveau cutané. Ces composés sont connus pour avoir des effets carcinogènes et immunogènes au niveau cutané.

Des solutions ont déjà été envisagées dans les traitements cosmétiques et thérapeutiques en protégeant les tissus par des composés avec des groupes soufrés qui se comportent comme des séquestrants de métaux lourds, comme les métalothionéines dans le brevet EP 0 557 042 A1 et les composés aminoacides avec des groupements soufrés dans la demande de brevet EP 0 914 815 A1.

La demande de brevet GB 233 3705 mentionne l'utilisation d'acide d'éthylène diamide disuccinique dans des compositions pour le traitement des irritations de la peau par les métaux lourds.

Par ailleurs le document EP-A-0 496 173 décrit des extraits de noix d'Alep contenant de l'acide ellagique en combinaison avec de l'acide gallique et des tanins hydrolysables pour prévenir les effets nocifs des radicaux libres. Ce document prévoit également une application en cosmétique comme filtre protecteur des ultra-violets B responsables du vieillissement cutané.

De nombreux brevets antérieurs couvrent essentiellement l'utilisation de l'acide ellagique pour ses propriétés dépigmentantes, de filtration des rayonnements ultra-violet, anticancéreuses et anti-inflammatoires.

Le problème posé est donc de protéger la peau contre les gaz, les métaux lourds, les composés organiques résidus de combustion et leurs effets délétères rencontrés dans la pollution urbaine, agissant de manière isolés ou combinés.

Il a maintenant été constaté de manière tout à fait surprenante que l'utilisation en application topique de l'acide ellagique, de ses sels, de ses complexes métalliques, de ses dérivés mono- ou polyéthers, de ses dérivés mono- ou polyacylés ainsi que de ses dérivés carbonates ou carbamates, dérivant des groupements hydroxyles permettait de protéger les matières kératiniques, la peau et les phanères, contre les effets délétères des gaz, des métaux lourds et des composés organiques résidus de combustion.

La demanderesse a découvert que l'acide ellagique permettait de préserver et de protéger les matières kératiniques, la peau et les phanères contre les effets nocifs de la pollution.

L'acide ellagique présente un intérêt majeur en tant que molécule active contre les effets délétères de la pollution sur la peau. Il a l'avantagé d'exercer un effet protecteur contre des polluants de nature différente à des concentrations faibles.

L'acide ellagique, également dénommé: 2,3,7,8-tétrahydroxy(1)-benzopyrano(5,4,3-cde)(1)benzopyran-5,10 dione est une molécule bien connue appartenant au groupe des polyphénols et présente dans le règne végétal. On pourra se reporter à la publication du Merck Index 20ème édition (1996), n° 3588.

On connaît par le document FR-A-1 478 523, un procédé de purification de l'acide ellagique ainsi que les acides ellagiques purifiés obtenus par un tel procédé.

L'acide ellagique présente la formule chimique suivante : 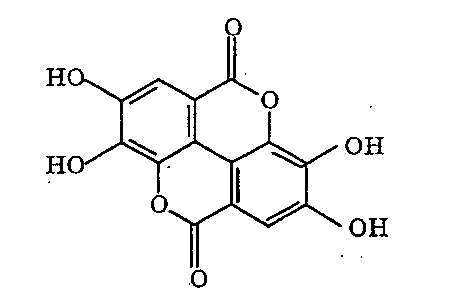 qui comporte quatre cycles accolés.

L'acide ellagique est disponible dans le commerce, notamment auprès de la Société Sigma, France.

La présente invention a pour objet une utilisation en application topique de l'acide ellagique, de ses sels, de ses complexes métalliques, de ses dérivés mono- ou polyéthers, de ses dérivés mono- ou polyacylés, ainsi que de ses dérivés carbonates ou carbamates, dérivant des groupements hydroxyles, comme agents cosmétiques anti-pollution.

On entend par agent cosmétique anti-pollution un agent qui protège la peau et les matières kératiniques de façon à prévenir, atténuer et/ou supprimer les effets délétères des gaz toxiques tels que l'ozone, des métaux et des composés organiques résidus de combustions.

L'acide ellagique et ses dérivés sont utilisés comme agents cosmétiques piégeurs de gaz toxiques et/ou comme agents cosmétiques chélateurs de métaux lourds et/ou comme agents cosmétiques permettant de prévenir les réactions d'hypersensibilité de contact due entre autres aux hydrocarbures aromatiques polycycliques.

La présente invention a également pour objet l'utilisation de l'acide ellagique et de ses dérivés, dans ou pour la préparation d'une composition cosmétique à application topique anti-polluante.

Dans le cadré de l'invention, les sels de l'acide ellagique comprennent en particulier les sels métalliques, notamment alcalins ou alcalino-terreux, tels que le sodium et le calcium, les sels d'amines tels que les sels de méthylglutamine, de diéthanolamine, de triéthanolamine, de choline, de bis-triéthylamine, les sels d'acides aminés, notamment les sels d'acides aminés basiques tels que l'arginine, la lysine et l'ornithine, les complexes métalliques comprennent en particulier des complexes métalliques avec le zinc et le cuivre et les dérivés mono- ou polyacylés comprennent en particulier des groupes acyles, saturés ou insaturés, ayant de 2 à 22 atomes de carbone. De préférence, ces groupes acyles correspondent aux acides acétique, palmitique, oléique, linoléique, linolénique, arachidonique, stéarique, brassidique, érucique, béhénique et (all Z)-5,8,11,14,17-eicosapentaénoïque. Les dérivés mono- ou polyéthers précités sont en particulier des dérivés alcoxy comprenant de 1 à 4 atomes de carbone, ou bien des dérivés de condensation de l'un ou de plusieurs groupes hydroxy de l'acide ellagique avec un sucre ou une chaîne de sucres. En particulier, il s'agit de l'acide 3-méthoxy ellagique ou des dérivés mono- ou polyéthers avec les sucres tels que le glucose, l'arabinose, le rhamnose et le galactose.

Les dérivés éthers ou acylés précités peuvent être obtenus par des procédés d'éthérification ou d'acylation des polyphénols bien connus de l'homme de l'art. Certains peuvent également être obtenus par extraction à partir de plantes.

Les compositions cosmétiques utilisées dans l'invention contiendront avantageusement de 0,001% à 10%, de préférence entre 0,01 et 5% en poids de l'acide ellagique, de ses sels, de ses complexes métalliques, de ses dérivés mono- ou polyéthers, mono- ou polyacylés et de ses dérivés carbonates ou carbamates, dérivant des groupements hydroxyles, par rapport au poids total de la composition.

Cette composition peut contenir en outre au moins un autre composé anti-pollution.

Celui-ci peut notamment être choisi parmi les anthocyanes et/ou ses dérivés, les composés contenant une fonction thio-éther, sulfoxydes ou sulfone, l'ergothionine et/ou ses dérivés, les agents chélateurs de métaux lourds comme par exemple les dérivés de l'acide N,N'-dibenzyl éthylène diamine N,N'-diacétique, les antioxydants, les extraits cellulaires de végétal de la famille Pontederiaceae.

La composition cosmétique utilisée dans l'invention peut contenir en outre un milieu cosmétiquement acceptable, qui est plus particulièrement constitué d'eau et/ou éventuellement de solvant organique cosmétiquement acceptable.

Ils peuvent être choisis dans le groupe constitué par les solvants organiques hydrophiles, les solvants amphiphiles, les solvants organiques lipophiles ou leurs mélanges.

Parmi les solvants organiques hydrophiles, on peut citer par exemple des mono-alcools inférieurs, linéaires ou ramifiés, ayant de 1 à 8 atomes de carbone comme l'éthanol, le propanol, le butanol, l'isopropanol, l'isobutanol, les polyéthylèneglycols ayant de 6 à 80 oxydes d'éthylène, les polyols tels que le propylèneglycol, l'isoprène glycol, le butylèneglycol, le glycérol, le sorbitol, les mono- ou dialkyles d'isosorbide dont les groupements alkyle ont de 1 à 5 atomes de carbone comme le diméthyl isosorbide, les éthers de glycol comme le diéthylène glycol mono-méthyle ou mono-éthyléther et les éthers de propylène glycol comme le dipropylène glycol méthyléther.

Comme solvants organiques amphiphiles, on peut citer des polyols tels que des dérivés de propylèneglycol (PPG), tels que les esters de polypropylèneglycol et d'acides gras, de PPG et d'alcools gras comme le PPG-23 oleyléther et le PPG-36 oléate.

Comme solvants organiques lipophiles, on peut citer par exemple les esters gras tels que l'adipate de diisopropyle, l'adipate de dioctyle, les benzoates d'alkyle.

Les solvants organiques sont choisis préférentiellement parmi les alcools mono- ou polyfonctionnels, les polyéthylène glycols éventuellement oxyéthylénés, les esters de polypropylène glycol, le sorbitol et ses dérivés, les dialkyls d'isosorbide, les éthers de glycol et des éthers de polypropylène glycol, les esters gras.

Les solvants organiques peuvent représenter de 5 à 98% du poids total de la composition.

Afin que les compositions utilisées dans l'invention soient plus agréables à utiliser, plus douces à l'application, plus nourrissantes et plus émolliantes, il est possible d'ajouter une phase grasse dans le milieu de ces compositions.

La phase grasse représente, de préférence, de 0 à 50% en poids total de la composition.

Cette phase grasse peut comporter une ou plusieurs huiles choisies de préférence dans le groupe constitué par :
- les silicones volatiles ou non-volatiles, linéaires, ramifiées ou cycliques, organo-modifiées ou non, hydrosolubles ou liposolubles,
- les huiles minérales telles que l'huile de paraffine et de vaseline,
- les huiles d'origine animale telles que le perhydrosqualène,
- les huiles d'origine végétale telles que l'huile d'amandes douces, l'huile d'avocat, l'huile de ricin, l'huile d'olive, l'huile de jojoba, l'huile de sésame, l'huile d'arachide, l'huile de macadamia, l'huile de pépins de raisin, l'huile de colza, l'huile de coprah,
- les huiles synthétiques telles que l'huile de Purcellin, les isoparaffines,
- les huiles fluorées et perfluorées,
- les esters d'acides gras tels que l'huile de Purcellin.

Elle peut aussi comporter comme matières grasses (un) ou plusieurs alcools gras, acides gras ou cires (paraffine, cire de polyéthylène, Carnauba, cire d'abeilles).

De façon connue, les compositions utilisées dans l'invention peuvent en outre contenir des adjuvants habituels dans le domaine cosmétique tels que les gélifiants et/ou épaississants classiques aqueux ou lipophiles, les actifs hydrophiles ou lipophiles, les conservateurs, des antioxydants, les parfums, les émulsionnants, les agents hydratants, les agents pigmentants, les dépigmentants, les agents kératolytiques, les vitamines, les émollients, les séquestrants, les tensio-actifs, les polymères, les agents alcalinisants ou acidifiants, les charges, les agents anti-radicaux libres, les céramides, les filtres solaires, notamment ultra-violets, les répulsifs pour insectes, les agents amincissants, les matières colorantes, les bactéricides, les anti-pelliculaires.

Les quantités de ces différents adjuvants sont celles classiquement utilisées dans les domaines considérés.

Bien entendu, l'homme de l'art veillera à choisir le ou les éventuels composés à ajouter à la composition selon l'invention, de manière telle que les propriétés avantageuses attachées intrinsèquement à la composition conforme à l'invention ne soient pas, ou substantiellement pas, altérées par l'addition envisagée.

Les compositions utilisées selon l'invention peuvent se présenter sous toutes les formes galléniques normalement utilisées pour une application topique, notamment sous forme d'une solution aqueuse, hydroalcoolique ou huileuse, d'une émulsion huile-dans-eau ou eau-dans-huile ou multiple, d'un gel aqueux ou huileux, d'un produit anhydre liquide, pâteux ou solide ou d'une dispersion d'huile dans une phase aqueuse à l'aide de sphérules, ces sphérules pouvant être des nanoparticules polymériques telles que les nanosphères et les nanocapsules, ou mieux, des vésicules lipidiques de type ionique et/ou non-ionique.

Les compositions utilisées dans la présente invention peuvent être plus ou moins fluides et avoir l'aspect d'une crème blanche ou colorée, d'une pommade, d'un lait, d'une lotion, d'un sérum, d'une pâte, d'une mousse ou d'un solide.

Elles peuvent éventuellement être appliquées sur la peau sous forme d'aérosol.

Elles peuvent également se présenter sous forme solide, et par exemple sous forme de stick.

Elles peuvent être utilisées comme produit de soin et/ou comme produit de maquillage.

Les compositions selon l'invention peuvent avoir un pH compris entre 3 et 8, et préférentiellement entre 5 et 7.

Un autre objet de l'invention consiste en un procédé de traitement cosmétique destiné à obtenir une protection de l'organisme contre les effets de la pollution, consistant à appliquer sur la peau une quantité cosmétiquement efficace de l'acide ellagique, de ses sels, de ses complexes, de ses dérivés mono- ou polyéthers, mono- ou polyacylés et de ses dérivés carbonates ou carbamates, dérivant des groupements hydroxyles.

Un autre procédé de traitement cosmétique selon l'invention destiné à obtenir une protection de l'organisme contre les effets de la pollution, consiste à appliquer sur la peau une composition cosmétique selon l'invention, telle que définie ci-dessus.

Les exemples qui suivent sont destinés à illustrer l'invention sans pour autant présenter un caractère limitatif.

### EXPERIMENTATION

### 1. Protection contre les effets de l'ozone

### Principe :

L'ozone a la capacité d'oxyder les constituants cellulaires en générant notamment des protéines carbonylées et des hydroperoxydes lipidiques. La quantification des hydroperoxydes lipidiques est un moyen de mesure du stress oxydatif induit par une exposition du tissu cutané à ce polluant. Une diminution de leur taux indique un effet protecteur de l'acide ellagique.

### Type cellulaire et culture :

L'étude a été réalisée sur une culture monocouche de kératinocytes humains issus de plasties chirurgicales. Les cellules sont ensemencées à J-3 en boîtes 48 puits à raison de 25000 cellules/cm² dans 500 µl de milieu de culture. Les incubations sont réalisées à 37°C, 5% de CO₂ en atmosphère humide.

### Pré-traitement des kératinocytes par l'acide ellagique (J-1) :

Les cellules ont été prétraitées 24 heures avec l'acide ellagique (100 µM final).

### Incorporation d'un marqueur de stress oxydant, DCFH-DA (2,7-dichlorofluorescine diacétate) :

Les hydropéroxydes constituent un marqueur du stress intracellulaire. Ils sont détectés et quantifiés au moyen d'une technique de fluorescence (Lebel C.P., Ischiropoulos H. and Bondy S.C. (1992) Evaluation of the probe 2,7-dichlorofluorescin as an indicator of Reactive Oxygen Species formation and oxydative stress. Chem. Res. Toxicol.; 5: 227-231).

En présence d'hydroperoxydes et des péroxydases intracellulaires, la DCFH est oxydée en 2, 7-dichlorofluorescéine (DCF) fluorescente.

Les cellules, prétraitées 24 heures par l'acide ellagique, sont alors lavées par du tampon phosphate salin (PBS) et mises en contact 30 minutes avec une solution de DCFH-DA (500 µl/puits), préparée dans le milieu de culture à la concentration de 320 µM.

### Exposition à l'ozone :

Les cellules sont de nouveau rincées avec du tampon PBS puis mises en contact avec une solution d'acide ellagique (100 µl/puits), préparée dans du PBS à la concentration de 200 µM. Elles sont ensuite exposées à l'ozone (10 ppm), en atmosphère humide, dans un incubateur régulé à 37°C.

### Mesures des hydropéroxydes lipidiques induits par l'ozone :

La formation de DCF fluorescente (filtres d'excitation à 485 nm et d'émission à 530 nm) résultant de la production d'hydroperoxydes, est mesurée après différents temps d'exposition à l'ozone : 0, 5, 10 et 20 minutes.

### Résultats :

Toxicité de l'ozone envers les kératinocytes humains en culture, en absence et en présence d'acide ellagique à la concentration de 200 µM., en fonction du temps d'exposition.

| **Fluorescence observée en présence d'acide ellagique exprimée en % par rapport aux témoins non protégés pour chaque temps** | | |
|---|---|---|
| **5 min. de contact** **% fluorescence observée** **+/- SEM** | **10 min. de contact** **% fluorescence observée** **+/- SEM** | **20 min. de contact** **% fluorescence observée +/- SEM** |
| 28,2 +/- 3,3 | 36,3 +/- 3,4 | 52,3 +/- 4,4 |

Pour chaque temps, les valeurs de fluorescence des témoins non protégés sont ramenées à 100%. Les résultats, en présence d'acide ellagique sont alors exprimés par rapport à cette valeur témoin. L'acide ellagique diminue de façon significative le stress induit par l'ozone. Cette protection est maximale dès 5 minutes d'exposition (chute de 71,8% du stress induit). Elle est toujours significative après 20 minutes d'exposition (chute de 47,7% du stress induit).

A partir d'un modèle biologique, in vitro, utilisant des kératinocytes humains en culture, nous avons montré :
- qu'un agent représentatif d'une catégorie de polluants atmosphériques comme l'ozone, entraîne dans nos conditions expérimentales l'apparition d'un stress important,
- que l'acide ellagique exerce un effet protecteur hautement significatif contre l'effet induit par ce polluant.

### 2. Protection contre la cytotoxicité des métaux lourds

### Principe :

Les métaux lourds tels que le cadmium, le nickel, le plomb, le mercure, etc., exercent un effet cytotoxique sur les cellules de différents organes dont la peau. La technique de mesure de la viabilité cellulaire par le test d'incorporation du rouge neutre a permis de mettre en évidence l'effet cyto-protecteur de l'acide ellagique contre la toxicité du cadmium.

### Ensemencement des cellules et conditions de culture :

L'étude a été réalisée sur une culture monocouche de kératinocytes humains issus de plasties chirurgicales. Les cellules sont ensemencées à J-3 en boîtes 96 puits à raison de 25000 cellules/cm² dans 100 µl de milieu de culture. Les incubations sont réalisées à 37°C en atmosphère humide et enrichies à 5% de CO₂.

### Traitement des cellules :

Initialement, les cellules sont traitées 24 heures par des concentrations croissantes (0, 10, 25, 50, 75, 100, 150 et 200 µM) de chlorure de cadmium (CdCl₂), de façon à déterminer sa cytotoxicité. Dans un deuxième temps, elles sont traitées également 24 heures par les mêmes concentrations de CdCl₂ en présence d'acide ellagique (200 et 100 µM, concentrations correspondant aux dose et demi-dose maximales d'acide ellagique, non cytotoxiques pour les cellules).

### Mesure de la viabilité cellulaire :

A la fin du traitement, la viabilité cellulaire est déterminée par le test d'incorporation de rouge neutre (POS 55/006) et lecture à 550 nm (réf : Borenfreund, E and Puerner, J.A (1984) A simple quantitative procedure using monolayer cultures for cytotoxicity assays. Tissue. Culture Methods; 9:7-9).

Les cellules sont rincées par du tampon PBS afin d'éliminer les solutions de traitement, puis incubées 3 heures à 37°C dans une solution de rouge neutre (100 µl) préparée à la concentration de 0,5 mg/ml dans le milieu de culture. Elles sont ensuite rincées par du tampon PBS puis fixées pendant une minute dans une solution de formol/calcium. Le rouge neutre est alors extrait par une solution d'éthanol/acide acétique (100 µl/puits). La quantité extraite est déterminée par lecture de la densité optique au spectrophotomètre à 550 nm.

La concentration de CdCl₂ induisant une chute de 50% de la viabilité cellulaire (CI-50) est ensuite calculée.

### Résultats:

Cytotoxicité du chlorure de cadmium envers les kératinocytes humains en culture, en absence et en présence d'acide ellagique à deux concentrations 100 et 200 µM (n=4).

| **CI.50 du chlorure de cadmium** | | |
|---|---|---|
| **sans acide ellagique** | **avec acide ellagique** | |
| Moyenne +/- SEM | Moyenne +/- SEM | |
| | **100 µM** | **200 µM** |
| **39 +/- 1,2 µM** | **99 +/- 2,9 µM** | **167 +/- 9,8 µM** |

Le chlorure de cadmium seul, présente une toxicité importante avec une CI-50 de 39 µM. En présence d'acide ellagique, la cytotoxicité du chlorure de cadmium diminue très fortement (ce qui correspond à une augmentation de la CI.50):
- à 100 µM d'acide ellagique, la cytotoxicité diminue d'un facteur 2,5.
- à 200 µM d'acide ellagique, la cytotoxicité diminue d'un facteur 4,3.

A partir d'un modèle biologique in vitro utilisant des kératinocytes humains en culture, nous avons montré :
- qu'un agent représentatif d'une catégorie de polluants atmosphériques (métaux lourds) comme le cadmium, entraîne dans nos conditions expérimentales une forte toxicité.
- que l'acide ellagique exerce un effet cytoprotecteur contre la toxicité de ce polluant.

### 3. Protection contre l'alkylation des nucléophiles induites par le métabolisme cutané des hydrocarbures aromatiques polycycliques (HAP)

### Principe :

Les hydrocarbures aromatiques polycycliques (HAP), adsorbés à la surface des particules et poussières véhiculées par l'air urbain, peuvent pénétrer le tissu cutané et y être biotransformés. Leur métabolisme hépatique, bien décrit dans la littérature, conduit à la formation de métabolites monohydroxylés (voie de détoxification), d'époxydes et diols époxydes (voie toxifiante). Il suit le même profil au niveau cutané. La voie toxifiante des époxydes et diols époxydes génère une alkylation des nucléophiles (protéines et l'ADN), qu'il est possible de mettre en évidence par une mesure de la fixation covalente à ces macromolécules en étudiant le métabolisme d'un HAP radiomarqué (¹⁴C benzo[a]pyrène). Après des expositions conséquentes ou répétées au polluant, sa toxicité potentielle peut conduire à une hypersensibilité de contact dans le cas de l'alkylation des protéines.

Le principe de l'étude a été de mettre en évidence un effet protecteur de l'acide ellagique vis-à-vis des entités réactives alkylantes produites par le métabolisme cutané de B [a]P radiomarqué en mesurant la radioactivité covalemment liée aux protéines.

### Type cellulaire étudié et culture :

L'étude a été réalisée sur une culture monocouche de kératinocytes humains issus de plasties chirurgicales. Les cellules sont ensemencées à J-3 en boîtes 6 puits à raison de 53000 cellules/cm². Les incubations sont réalisées à 37°C en atmosphère humide et enrichies à 5% de CO₂.

### Traitement des cellules :

Les cellules sont mises en contact 24 heures avec le ¹⁴C B[a]P (20 µM) et l'acide ellagique Sigma, France (100 µM), tous deux coincubés. Après ce contact, les cellules ont été lavées par du tampon, PBS, grattées dans le même tampon (0,5 ml), puis congelées dans l'azote liquide et conservées à -80°C jusqu'à l'analyse.

### Mesure de la fixation covalente aux protéines cellulaires :

La fixation covalente aux protéines cellulaires est mesurée selon le protocole de Hoellinger et al., adapté à une méthode de filtration en microplaques 96 puits (H. Hoellinger, M. Sonnier, J. Gray, T.A. Connors, J. Pichon and N.H. NAM. In vitro covalent binding of cismethrin, bioresmethrin and their common alcohol to hepatic proteins. Toxicol. Appl. Pharmacol., 1985, 77, 11-18).

Une fraction aliquote des protéines cellulaires (200 µl) est précipitée dans les puits de la microplaque par de l'acide perchlorique à 10% (50 µl). La plaque est ensuite transférée sur un système de filtration sous vide où le contenu des puits est aspiré et les protéines retenues sur les membranes filtrantes, lavées par les solvants acétate d'éthyle (3 x 200 µl), acétone (200 µl), éthanol (200 µl) et tampon PBS (200 µl). Cette série de lavages ayant pour but d'éliminer des protéines toute la radioactivité non fixée de façon covalente, les filtres de la microplaque sont récupérés individuellement et les protéines retenues à leur surface sont digérées dans la soude 1N (400 µl) 24 heures à 37°C. Des prélèvements sont ensuite effectués pour doser les protéines et compter la radioactivité de la solution.

Les résultats sont exprimés en nmol de B[a]P fixées par mg de protéines.

### Résultats:

Fixation covalente du Benzo(a)pyrene sur des kératinocytes humains en culture en absence et en présence d'acide ellagique à la concentration de 100µM (n=4).

| **Fixation covalente du B(a)P** | |
|---|---|
| **sans acide ellagique** en nmoles de B(a)P par mg de protéine | **avec acide ellagique** en nmoles de B(a)P par mg de protéine |
| Moyenne +/- SEM **0,28 +/- 0,07 µM** | Moyenne +/- SEM **0,09 +/- 0,02 µM** |

Le Benzo(a)pyrène seul à la concentration de 20 µM présente une réactivité importante avec une fixation covalente aux protéines kératinocytaires de 0,28 nmoles de B(a)P par mg de protéine. En présence d'acide ellagique, la réactivité du B(a)P diminue très fortement (réduction de la réactivité d'un facteur 3,1).

A partir d'un modèle biologique in vitro utilisant une cellule d'origine épithéliale humaine (kératinocyte/peau), nous avons montré:
- qu'un agent représentatif d'une catégorie de polluants atmosphériques (HAP) comme le benzo[a]pyrène, entraîne dans nos conditions expérimentales une toxicité potentielle liée à ses capacités métaboliques à produire des entités alkylantes,
- que l'acide ellagique exerce un effet protecteur contre cette forme de toxicité induite par de tels polluants.

### EXEMPLES DE FORMULATION

### Exemple 1: Selon les techniques usuelles de préparation, on mélange les constituants ci-dessous pour préparer une émulsion.

| **COMPOSITION POUR L'APPLICATION TOPIQUE** | |
|---|---|
| sel sodique d'acide ellagique | 5 g |
| polyéthylèneglycol oxyéthyléné par 50 moles d'oxyde d'éthylène | 3 g |
| monodiglycérylstéarate | 3 g |
| huile de vaseline | 24 g |
| alcool cétylique | 5 g |
| eau qsp | 100 g |

### Exemple 2: De la même manière, on prépare une émulsion selon une technique classique, à partir des composés suivants:

| | |
|---|---|
| sel de diéthènolamine d'acide ellagique | 1 g |
| octylpalmitate | 10 g |
| glycérylisostéarate | 4 g |
| huile de purcellin | 23 g |
| vitamine E | 1 g |
| glycérol | 3 g |
| eau qsp | 100 g |

### Exemple 3: De la même manière, on prépare une émulsion selon une technique classique, à partir des composés suivants:

| | |
|---|---|
| acide 3-méthoxy ellagique | 0,01 g |
| octylpalmitate | 10 g |
| glycérylisostéarate | 4 g |
| huile de vaseline | 20 g |
| sorbitol | 2 g |
| vitamine E | 1 g |
| glycérol | 3 g |
| eau qsp | 100 g |

### Exemple 4: De la même manière, on prépare une émulsion selon une technique classique, à partir des composés suivants:

| | |
|---|---|
| acide ellagique monoacétylé | 0,5 g |
| octylpalmitate | 10 g |
| glycérylisostéarate | 4 g |
| huile de vaseline | 24 g |
| vitamine E | 1 g |
| glycérol | 3 g |
| eau qsp | 100 g |

### Exemple 5: A partir des constituants ci-dessous, on formule la composition suivante:

| | |
|---|---|
| sel calcique d'acide ellagique | 1,5 g |
| huile de jojoba | 13 g |
| parabenzoxy benzoate de méthyle et d'isopropyle | 0,05 g |
| sorbate de potassium | 0,3 g |
| cyclopentadimethylsiloxane | 10 g |
| alcool stéarylique | 1 g |
| acide stéarique | 4 g |
| stéarate de polyéthylèneglycol | 3 g |
| vitamine E | 1 g |
| glycérol | 3 g |
| eau qsp | 100 g |

### Exemple 6: A partir des constituants ci-dessous, on formule la composition suivante:

| | |
|---|---|
| acide ellagique compléxé avec le zinc | 1 g |
| huile de jojoba | 13 g |
| parabenzoxy benzoate de méthyle et d'isopropyle | 0,05 g |
| sorbate de potassium | 0,3 g |
| cyclopentadimethylsiloxane | 10 g |
| alcool stéarylique | 1 g |
| acide N,N'-di-(3-hydroxybenzyle)-éthylène diamine N,N'-diacétique | 0,01 g |
| acide stéarique | 4 g |
| stéarate de polyéthylèneglycol | 3 g |
| vitamine E | 1 g |
| glycérol | 3 g |
| eau qsp | 100 g |

### Exemple 7: A partir des constituants ci-dessous, on formule la composition suivante:

| | |
|---|---|
| sel de choline d'acide ellagique | 0,5 g |
| huile de jojoba | 13 g |
| parabenzoxy benzoate de méthyle et d'isopropyle | 0,05 g |
| sorbate de potassium | 0,3 g |
| cyclopentadimethylsiloxane | 10 g |
| alcool stéarylique | 1 g |
| acide stéarique | 4 g |
| extrait cellulaires de jacinthe d'eau | 0,05 g |
| stéarate de polyéthylèneglycol | 3 g |
| vitamine E | 1 g |
| glycérol | 3 g |
| eau qsp | 100 g |

L'extrait cellulaire de jacinthe d'eau (*Eichhornia crassipes*) a été obtenu par ce procédé: 12 pieds de jacinthe d'eau ont été lavés à l'eau puis grossièrement essorés. Passés en broyeur à couteau (robot coupe), on a obtenu 700 g de broyat. Ajout de 700 ml d'H₂O, puis 300 ml d'H₂O MilliQ. Nouveau passage en robot coupe 5 minutes, centrifugation 20'/8000 G filtration Whatmann GFD puis GFF et lyophilisation : 5,43 g de lyophilisat sont ainsi obtenus.

## Revendications

1. Utilisation cosmétique en application topique de l'acide ellagique, de ses sels, de ses complexes métalliques, de ses dérivés mono- ou poly-éthers, mono- ou poly-acylés et de ses dérivés carbonates ou carbamates dérivant des groupements hydroxyles comme agent de protection de la peau et des matières bératiniques de façon à prévenir, atténuer et/ou supprimer les effets délétères des gaz toxiques tels que l'ozone, des métaux et des composés organiques résidus de combustion.

2. Utilisation cosmétique en application topique de l'acide ellagique, de ses sels, de ses complexes métalliques, de ses dérivés mono- ou poly-éthers, mono- ou poly-acylés et de ses dérivés carbonates ou carbamates dérivant des groupements hydroxyles comme agent piégeur de gaz toxiques et/ou comme agent chélateur de métaux lourds et/ou comme agent permettant d'éviter l'hypersensibilité de contact due aux hydrocarbures aromatiques polycycliques.

3. Utilisation cosmétique de l'acide ellagique de ses sels, de ses complexes métalliques, de ses dérivés mono-ou poly-éthers, mono-ou poly-acylés et de ses dérivés carbonates ou carbamates dérivant des groupements hydroxyles dans une composition à application topique de protection de la peau et des matières kératiniques de façon à prévenir, atténuer et/ou supprimer les effets délétères des gaz toxiques tels que l'ozone, des métaux et des composés organiques résidus de combustion.

4. Utilisation de l'acide ellagique de ses sels, de ses complexes métalliques, de ses dérivés mono-ou poly-éthers, mono-ou poly-acylés et de ses dérivés carbonates ou carbamates dérivant des groupements hydroxyles pour la préparation d'une composition à application topique de protection de la peau et des matières kératiniques de façon à prévenir, atténuer et/ou supprimer les effets délétères des gaz toxiques tels que l'ozone, des métaux et des composés organiques résidus de combustion.

5. Utilisation selon l'une des revendications 1 à 4, **caractérisée en ce que** les sels de l'acide ellagique comprennent un sel métallique, en particulier alcalin ou alcalino-terreux tel que le sodium et le calcium, les sels d'amines tels que les sels de méthylglutamine, de diéthanolamine, de triéthanolamine, de choline, de bis-triéthylamine, les sels d'acides aminés notamment les sels d'acides aminés basiques tels que l'arginine, la lysine et l'ornithine, les complexes métalliques avec le zinc et le cuivre, et les dérivés mono- ou poly-acylés comprennent des groupes acyles, saturés ou insaturés, ayant de 2 à 22 atomes de carbone, de préférence ces groupes acyles correspondent aux acides acétique, palmitique, oléique, linoléique, linolénique, arachidonique, stéarique, brassidique, érucique, béhénique et (all Z)-5,8,11,14,17-eicosapentoènoïque; les dérivés mono-ou poly-éthers précités sont des dérivés alcoxy comprenant de 1 à 4 atomes de carbone, ou bien des dérivés de condensation de l'un ou de plusieurs groupes hydroxy de l'acide ellagique avec un sucre ou une chaîne de sucres, en particulier l'acide 3-méthoxy ellagique ou ses dérivés mono- ou poly-éthers avec les sucres tels que le glucose, l'arabinose, le rhamnose et le galactose.

6. Utilisation selon la revendication 3 à 5, **caractérisée en ce que** ladite composition cosmétique anti-polluante contient de 0,001% à 10% et de préférence de 0,01 à 5% en poids d'acide ellagique, de ses sels, de ses complexes métalliques, de ses dérivés mono- ou poly-éthers, mono-ou poly-acylés et de ses dérivés carbonates ou carbamates dérivant des groupements hydroxyles, par rapport au poids total de la composition.

7. Utilisation selon l'une des revendications 3 à 6, **caractérisée en ce que** ladite composition contient en outre au moins un autre composé anti-pollution.

8. Utilisation selon la revendication 7, **caractérisée en ce que** ledit composé est choisi parmi le groupe constitué par les anthocyanes et/ou ses dérivés, les composés contenant une fonction thio-éther, sulfoxydes ou sulfone, l'ergothionine et/ou ses dérivés, les agents chélateurs de métaux lourds comme par exemple les dérivés de l'acide N,N'-dibenzyl éthylène diamine N,N'-diacétique, les antioxydants, les extraits cellulaires de végétal de la famille Pontederiaceae.

9. Utilisation selon l'une quelconque des revendications 1 à 8, **caractérisée en ce que** ladite composition peut contenir en outre un milieu cosmétiquement acceptable constitué d'eau et/ou éventuellement de solvant organique cosmétiquement acceptable.

10. Utilisation selon la revendication 9, **caractérisée en ce que** le solvant organique est choisi dans le groupe constitué par les solvants organiques hydrophiles, les solvants amphiphile, les solvants organiques lipophiles ou leurs mélanges.

11. Utilisation selon l'une quelconque des revendications 9 à 10, **caractérisée en ce que** les solvants organiques sont choisis préférentiellement parmi les alcools mono- ou polyfonctionnels, les polyéthylène glycols éventuellement oxyéthylénés, les esters de polypropylène glycol, le sorbitol et ses dérivés, les dialkyls d'isosorbide, les éthers de glycol et des éthers de polypropylène glycol, les esters gras.

12. Utilisation selon la revendication 9 ou 11, **caractérisée en ce que** le ou les solvants organiques représentent de 5 à 98% du poids total de la composition.

13. Utilisation selon l'une quelconque des revendications 1 à 12, **caractérisée en ce que** ladite composition comprend en outre au moins une phase grasse.

14. Utilisation selon la revendication 13, **caractérisée en ce que** la phase grasse représente de 0 à 50% du poids total de la composition.

15. Utilisation selon l'une quelconque des revendications 1 à 14, **caractérisée par le fait que** ladite composition contient en outre au moins un additif choisi dans le groupe constitué par les gélifiants et/ou épaississants classiques aqueux ou lipophiles, les actifs hydrophiles ou lipophiles, les conservateurs, des antioxydants, les parfums, les émulsionnants, les agents hydratants, les agents pigmentants, les dépigmentants, les agents kératolytiques, les vitamines, les émollients, les séquestrants, les tensio-actifs, les polymères, les agents alcalinisants ou acidifiants, les charges, les agents anti-radicaux libres, les céramides, les filtres solaires, notamment ultra-violets, les répulsifs pour insectes, les agents amincissants, les matières colorantes, les bactéricides, les anti-pelliculaires.

16. Utilisation selon l'une quelconque des revendications 1 à 15, **caractérisée en ce que** ladite composition se présente sous forme d'une solution aqueuse; hydroalcoolique ou huileuse, d'une émulsion huile-dans-eau ou eau-dans-huile ou multiple, d'un gel aqueux ou huileux, d'un produit anhydre liquide, pâteux ou solide ou d'une dispersion d'huile dans une phase aqueuse à l'aide de sphérules.

17. Utilisation selon l'une quelconque des revendications 1 à 16, **caractérisée en ce que** ladite composition a l'aspect d'une crème blanche ou colorée, d'une pommade, d'un lait, d'une lotion, d'un sérum, d'une pâte, d'une mousse ou d'un solide.

18. Procédé cosmétique de protection de l'organisme contre les effets de la pollution, **caractérisé en ce qu'**il consiste à appliquer sur la peau une composition telle que définie dans l'une quelconque des revendications 1 à 17.

## Patentansprüche

1. Kosmetische Verwendung unter topischer Anwendung von Ellagsäure, ihrer Salze, ihrer Metallkomplexe, ihrer Mono- oder Polyetherderivate, ihrer mono- oder polyacylierten Derivate und ihrer Carbonat- oder Carbamatderivate, die sich von den Hydroxygruppen ableiten, als Mittel zum Schutz der Haut und der Keratinmaterialien, um die schädlichen Wirkungen toxischer Gase, wie Ozon, von Metallen und von organischen Verbrennungsrückständen zu verhindern, abzuschwächen und/oder zu beseitigen.

2. Kosmetische Verwendung unter topischer Anwendung von Ellagsäure, ihrer Salze, ihrer Metallkomplexe, ihrer Mono- oder Polyetherderivate, ihrer mono- oder polyacylierten Derivate und ihrer Carbonat- oder Carbamatderivate, die sich von den Hydroxygruppen ableiten, als kosmetisches Mittel, das toxische Gase einfängt, und/oder als Mittel, das mit Schwermetallen Chelatkomplexe bildet, und/oder als Mittel, das es ermöglicht, die Kontaktüberempfindlichkeit, die durch polycyclische aromatische Kohlenwasserstoffe verursacht wird, zu vermeiden.

3. Kosmetische Verwendung von Ellagsäure, ihrer Salze, ihrer Metallkomplexe, ihrer Mono- oder Polyetherderivate, ihrer monooder polyacylierten Derivate und ihrer Carbonat- oder Carbamatderivate, die sich von den Hydroxygruppen ableiten, in einer Zusammensetzung, die topisch angewendet wird, zum Schutz der Haut und der Keratinmaterialien, um die schädlichen Wirkungen toxischer Gase, wie Ozon, von Metallen und von organischen Verbrennungsrückständen zu verhindern, abzuschwächen und/oder zu beseitigen.

4. Verwendung von Ellagsäure, ihrer Salze, ihrer Metallkomplexe, ihrer Mono- oder Polyetherderivate, ihrer mono- oder polyacylierten Derivate und ihrer Carbonat- oder Carbamatderivate, die sich von den Hydroxygruppen ableiten, für die Herstellung einer Zusammensetzung, die topisch angewendet wird, zum Schutz der Haut und der Keratinmaterialien, um die schädlichen Wirkungen toxischer Gase, wie Ozon, von Metallen und von organischen Verbrennungsrückständen zu verhindern, abzuschwächen und/oder zu beseitigen.

5. Verwendung nach einem der Ansprüche 1 bis 4, **dadurch gekennzeichnet, dass** die Salze der Ellagsäure Metallsalze, insbesondere Salze von Alkalimetallen oder Erdalkalimetallen, wie Natrium und Calcium, die Salze von Aminen, wie die Salze von Methylglutamin, Diethanolamin, Triethanolamin, Cholin, Bistriethylamin, die Salze von Aminosäuren, insbesondere die Salze basischer Aminosäuren, wie Arginin, Lysin und Ornithin, die Metallkomplexe mit Zink und Kupfer umfassen, dass die monooder polyacylierten Derivate gesättigte oder ungesättigte Acylgruppen umfassen, die 2 bis 22 Kohlenstoffatome aufweisen, wobei diese Acylgruppen vorzugsweise der Essigsäure, Palmitinsäure, Ölsäure, Linolsäure, Linolensäure, Arachidonsäure, Stearinsäure, Brassidinsäure, Erucasäure, Behensäure und (all-Z)-5,8,11,14,17-Eicosapentaensäure entsprechen, und dass die oben erwähnten Mono- oder Polyetherderivate Alkoxyderivate, die 1 bis 4 Kohlenstoffatome aufweisen, oder Derivate der Kondensation einer oder mehrerer Hydroxygruppen der Ellagsäure mit einem Zucker oder einer Zuckerkette sind, insbesondere die 3-Methoxyellagsäure oder ihre Mono- oder Polyetherderivate mit Zuckern wie Glucose, Arabinose, Rhamnose und Galactose.

6. Verwendung nach einem der Ansprüche 3 bis 5, **dadurch gekennzeichnet, dass** die vor Umweltbelastungen schützende kosmetische Zusammensetzung 0,001 bis 10 Gew.-% und vorzugsweise 0,01 bis 5 Gew.-% Ellagsäure, ihrer Salze, ihrer Metallkomplexe, ihrer Mono- oder Polyetherderivate, ihrer mono- oder polyacylierten Derivate und ihrer Carbonat- oder Carbamatderivate, die sich von den Hydroxygruppen ableiten, bezogen auf das Gesamtgewicht der Zusammensetzung, enthält.

7. Verwendung nach einem der Ansprüche 3 bis 6, **dadurch gekennzeichnet, dass** die Zusammensetzung außerdem mindestens eine weitere vor Umweltbelastungen schützende Verbindung enthält.

8. Verwendung nach Anspruch 7, **dadurch gekennzeichnet, dass** diese Verbindung aus der Gruppe ausgewählt wird, die aus den Anthocyanen und/oder deren Derivaten, den Verbindungen, die ein Thioethergruppe enthalten, Sulfoxiden oder Sulfonen, Ergothionin und/oder seinen Derivaten, Mitteln, die mit Schwermetallen Chelatkomplexe bilden, wie z.B. den Derivaten der N,N'-Dibenzylethylendiamin-N,N'-diessigsäure, den Antioxidantien, den pflanzlichen Zellextrakten von Pflanzen aus der Familie Pontederiaceae besteht.

9. Verwendung nach einem der Ansprüche 1 bis 8, **dadurch gekennzeichnet, dass** die Zusammensetzung außerdem ein kosmetisch akzeptables Medium enthalten kann, das aus Wasser und/ oder gegebenenfalls kosmetisch akzeptablem organischem Lösemittel besteht.

10. Verwendung nach Anspruch 9, **dadurch gekennzeichnet, dass** das organische Lösemittel aus der Gruppe ausgewählt wird, die aus den hydrophilen organischen Lösemitteln, den amphiphilen Lösemitteln, den lipophilen organischen Lösemitteln und ihren Gemischen besteht.

11. Verwendung nach einem der Ansprüche 9 und 10, **dadurch gekennzeichnet, dass** die organischen Lösemittel vorzugsweise unter den ein- oder mehrwertigen Alkoholen, den Polyethylenglykolen, die gegebenenfalls ethoxyliert sind, den Polypropylenglykolestern, Sorbit und seinen Derivaten, den Dialkylisosorbiden, den Glykolethern und den Polypropylenglykolethern, den Fettestem ausgewählt werden.

12. Verwendung nach Anspruch 9 oder 11, **dadurch gekennzeichnet, dass** das oder die organischen Lösemittel 5 bis 98 % des Gesamtgewichts der Zusammensetzung ausmachen.

13. Verwendung nach einem der Ansprüche 1 bis 12, **dadurch gekennzeichnet, dass** die Zusammensetzung außerdem mindestens eine Fettphase enthält.

14. Verwendung nach Anspruch 13, **dadurch gekennzeichnet, dass** die Fettphase 0 bis 50 % des Gesamtgewichts der Zusammensetzung ausmacht.

15. Verwendung nach einem der Ansprüche 1 bis 14, **dadurch gekennzeichnet, dass** die Zusammensetzung außerdem mindestens einen Zusatzstoff enthält, der aus der Gruppe ausgewählt wird, die aus den herkömmlichen wäßrigen oder lipophilen Gelbildnern und/oder Verdickungsmitteln, den hydrophilen oder lipophilen Wirkstoffen, den Konservierungsmitteln, den Antioxidantien, den Parfüms, den Emulgatoren, den Hydratisierungsmitteln, den Pigmentierungsmitteln, den Depigmentierungsmitteln, den Keratolytika, den Vitaminen, den Emollientien, den Maskierungsmitteln, den grenzflächenaktiven Stoffen, den Polymeren, den alkalisch machenden Mitteln oder Säuerungsmitteln, den Füllstoffen, den Mitteln gegen freie Radikale, den Ceramiden, den Sonnenschutzfiltern, insbesondere Ultraviolett-Filtern, den Repellentien gegen Insekten, den schlank machenden Mitteln, den Farbmitteln, den Bakteriziden, den Antischuppenmitteln besteht.

16. Verwendung nach einem der Ansprüche 1 bis 15, **dadurch gekennzeichnet, dass** die Zusammensetzung in Form einer wäßrigen, wäßrig-alkoholischen oder öligen Lösung, einer Öl-in-Wasser-Emulsion, Wasser-in-Öl-Emulsion oder multiplen Emulsion, eines wäßrigen oder öligen Gels, eines flüssigen, pastösen oder festen wasserfreien Produkts oder einer Dispersion eines Öls mit Hilfe von Kügelchen in einer wäßrigen Phase vorliegt.

17. Verwendung nach einem der Ansprüche 1 bis 16, **dadurch gekennzeichnet, dass** die Zusammensetzung das Aussehen einer weißen oder farbigen Creme, einer Pomade, einer Milch, einer Lotion, eines Serums, einer Paste, eines Schaumes oder eines Feststoffs hat.

18. Kosmetisches Verfahren zum Schutz des Organismus vor der Wirkung schädlicher Umwelteinflüsse, **dadurch gekennzeichnet, dass** es darin besteht, eine wie in einem der Ansprüche 1 bis 17 definierte Zusammensetzung auf die Haut aufzutragen.

## Claims

1. Use as a cosmetic agent in topical application of ellagic acid, its salts, its metal complexes, its monoether or polyether, monoacyl or polyacyl derivatives and its carbonate or carbamate derivatives, derived from the hydroxyl groups that protects the skin and keratin materials so as to prevent, attenuate and/or suppress the deleterious effects of toxic gases such as ozone, metals and organic compounds that are combustion residues.

2. Use as a cosmetic agent for trapping toxic gases and/or as a heavy-metal-chelating agent and/or as an agent for preventing the contact hypersensitivity caused by polycyclic aromatic hydrocarbons in topical application of ellagic acid, its salts, its metal complexes, its monoether or polyether, monoacyl or polyacyl derivatives and its carbonate or carbamate derivatives, derived from the hydroxyl groups.

3. Cosmetic use of ellagic acid, its salts, its metal complexes, its monoether or polyether, monoacyl or polyacyl derivatives and its carbonate or carbamate derivatives, derived from the hydroxyl groups in that protects the skin and keratin materials so as to prevent, attenuate and/or suppress the deleterious effects of toxic gases such as ozone, metals and organic compounds that are combustion residues.

4. Use of ellagic acid, its salts, its metal complexes, its monoether or polyether, monoacyl or polyacyl derivatives and its carbonate or carbamate derivatives, derived from the hydroxyl groups for the preparation of a composition for topical application that protects the skin and keratin materials so as to prevent, attenuate and/or suppress the deleterious effects of toxic gases such as ozone, metals and organic compounds that are combustion residues.

5. Use according to one of Claims 1 to 4, **characterized in that** the ellagic acid salts comprise a metal salt, in particular of an alkali metal or alkaline-earth metal, such as sodium and calcium, the amine salts such as the methylglutamine, diethanolamine, triethanolamine, choline and bis-triethylamine salts, the amino acid salts, especially the salts of basic amino acids such as arginine, lysine and ornithine, the metal complexes with zinc and copper and the monoacyl or polyacyl derivatives comprising saturated or unsaturated acyl groups containing from 2 to 22 carbon atoms. Preferably, these acyl groups correspond to acetic acid, palmitic acid, oleic acid, linoleic acid, linolenic acid, arachidonic acid, stearic acid, brassidic acid, erucic acid, behenic acid and (all Z)-5,8,11,14,17-eicosapentaenoic acid. The abovementioned monoether or polyether derivatives are alkoxy derivatives containing from 1 to 4 carbon atoms, or derivatives of condensation of one or more hydroxyl groups of ellagic acid with a sugar or a chain of sugars, in particular 3-methoxyellagic acid or its monoether or polyether derivatives with sugars such as glucose, arabinose, rhamnose and galactose.

6. Use according to Claims 3 to 5, **characterized in that** said antipollution cosmetic composition contains from 0.001% to 10% and preferably between 0.01% and 5% by weight of ellagic acid, its salts, its metal complexes, its monoether or polyether, monoacyl or polyacyl derivatives and its carbonate or carbamate derivatives, derived from the hydroxyl groups, relative to the total weight of the composition.

7. Use according to one of Claims 3 to 6, **characterized in that** said composition also contains at least one other antipollution compound.

8. Use according to Claim 7, **characterized in that** said compound is chosen from the group consisting of anthocyans and/or derivatives thereof, compounds containing a thioether function, sulphoxides or sulphones, ergothionine and/or its derivatives, heavy-metal-chelating agents such as, for example, N,N'-dibenzylethylenediamine-N,N'-di-acetic acid derivatives, antioxidants, and cell extracts of plants from the Pontederiacea family.

9. Use according to any one of Claims 1 to 8, **characterized in that** said composition may also contain a cosmetically acceptable medium consisting of water and/or optionally of a cosmetically acceptable organic solvent.

10. Use according to Claim 9, **characterized in that** the organic solvent is chosen from the group consisting of hydrophilic organic solvents, amphiphilic solvents and lipophilic organic solvents, or mixtures thereof.

11. Use according to any one of Claims 9 to 10, **characterized in that** the organic solvents are preferably chosen from monofunctional or polyfunctional alcohols, optionally oxyethylenated polyethylene glycols, polypropylene glycol esters, sorbitol and its derivatives, dialkyl isosorbides, glycol ethers, polypropylene glycol ethers and fatty esters.

12. Use according to Claim 9 or 11, **characterized in that** the organic solvent(s) represent(s) from 5% to 98% of the total weight of the composition.

13. Use according to any one of Claims 1 to 12, **characterized in that** said composition also comprises at least one fatty phase.

14. Use according to Claim 13, **characterized in that** the fatty phase represents from 0 to 50% relative to the total weight of the composition.

15. Use according to any one of Claims 1 to 14, **characterized in that** said composition also contains at least one additive chosen from the group consisting of standard aqueous or lipophilic gelling agents and/or thickeners, hydrophilic or lipophilic active agents, preserving agents, antioxidants, fragrances, emulsifiers, moisturizers, pigmenting agents, depigmenting agents, keratolytic agents, vitamins, emollients, sequestering agents, surfactants, polymers, acidifying or basifying agents, fillers, free-radical scavengers, ceramides, sunscreens, especially ultra-violet screening agents, insect repellents, slimming agents, dyestuffs, bactericides and antidandruff agents.

16. Use according to any one of Claims 1 to 15, **characterized in that** said composition is in the form of an aqueous, aqueous-alcoholic or oily solution, an oil-in-water or water-in-oil or multiple emulsion, an aqueous or oily gel, a liquid, pasty or solid anhydrous product or a dispersion of oil in an aqueous phase using spherules.

17. Use according to any one of Claims 1 to 16, **characterized in that** said composition has the appearance of a white or coloured cream, an ointment, a milk, a lotion, a serum, a paste, a mousse or a solid.

18. Cosmetic process for protecting the body against the effects of pollution, **characterized in that** it consists in applying to the skin a composition as defined in any one of Claims 1 to 17.
